# NOUVEAU FASCICULE DE BREVET EUROPEEN

(11) **EP 2 837 936 B2**
(45) Date de publication et mention de la décision concernant l'opposition: **25.11.2020**
(45) Mention de la délivrance du brevet: 11.10.2017
(21) Numéro de dépôt: 14180044.1
(22) Date de dépôt: 06.08.2014
(51) Int. Cl.: G01N 30/72, G01N 33/46, G01N 30/16

(54) **Procédé d'analyse d'un bouchon en liege pour la présence de 2,4,6-trichloroanisole et dispositif pour sa mise en oeuvre**
Analyseverfahren eines Naturkorkens zur Ermittlung der Präsenz von 2,4,6-Trichloranisol, und Vorrichtung zur Umsetzung dieses Verfahrens
Method for analysing a cork stopper for the presence of 2,4,6-trichloroanisole and device for implementing same

(30) Priorité: 13.08.2013 FR 1357968
(43) Date de publication de la demande: 18.02.2015
(73) Titulaire: Cevaqoe Invest, 31170 Tournefeuille (FR)
(72) Inventeur: Riboulet, Jean-Michel, 31170 TOURNEFEUILLE (FR); Reis Alves, Luiz Armando dos, 4535-311 PACOS DE BRANDAO (PT)
(74) Mandataire: Ipside

(56) Documents cités:
- US-A1- 2005 092 112
- US-A1- 2005 092 112
- US-A1- 2009 038 374
- LORENZO C ET AL: "Non-destructive method to determine halophenols and haloanisoles in cork stoppers by headspace sorptive extraction", JOURNAL OF CHROMATOGRAPHY, ELSEVIER SCIENCE PUBLISHERS B.V, NL, vol. 1114, no. 2, 12 mai 2006 (2006-05-12) , pages 250-254, XP024967450, ISSN: 0021-9673, DOI: 10.1016/J.CHROMA.2006.02.037 [extrait le 2006-05-12]
- PIZARRO ET AL: "Optimisation of a microwave-assisted extraction method for the simultaneous determination of haloanisoles and halophenols in cork stoppers", JOURNAL OF CHROMATOGRAPHY, ELSEVIER SCIENCE PUBLISHERS B.V, NL, vol. 1149, no. 2, 26 avril 2007 (2007-04-26), pages 138-144, XP022047969, ISSN: 0021-9673, DOI: 10.1016/J.CHROMA.2007.03.055
- RIU M ET AL: "Quantification of chloroanisoles in cork using headspace solid-phase microextraction and gas chromatography with electron capture detection", JOURNAL OF CHROMATOGRAPHY, ELSEVIER SCIENCE PUBLISHERS B.V, NL, vol. 1107, no. 1-2, 24 février 2006 (2006-02-24), pages 240-247, XP024968177, ISSN: 0021-9673, DOI: 10.1016/J.CHROMA.2005.12.063 [extrait le 2006-02-24]
- Isabel Márquez-Sillero ET AL: "Headspace-multicapillary column-ion mobility spectrometry for the direct analysis of 2,4,6-trichloroanisole in wine and cork samples", Journal of Chromatography A, vol. 1265, 1 November 2012 (2012-11-01), pages 149-154, XP055373775, AMSTERDAM, NL ISSN: 0021-9673, DOI: 10.1016/j.chroma.2012.09.087
- M. P. Martí ET AL: "Fast screening method for determining 2,4,6-trichloroanisole in wines using a headspace?mass spectrometry (HS?MS) system and multivariate calibration", ANALYTICAL AND BIOANALYTICAL CHEMISTRY, vol. 376, no. 4, 1 June 2003 (2003-06-01), pages 497-501, XP055373791, DE ISSN: 1618-2642, DOI: 10.1007/s00216-003-1940-z
- Natalia Campillo ET AL: "Purge-and-trap preconcentration system coupled to capillary gas chromatography with atomic emission detection for 2,4,6-trichloroanisole determination in cork stoppers and wines", Journal of Chromatography A, vol. 1061, no. 1, 1 December 2004 (2004-12-01), pages 85-91, XP055373801, AMSTERDAM, NL ISSN: 0021-9673, DOI: 10.1016/j.chroma.2004.11.005
- LORENZO, C. et al.: "Non-destructive method to determine halophenols and haloanisoles in cork stoppers by headspace sorptive extraction", Journal of Chromatography A, vol. 1114, no. 2, 12 May 2006 (2006-05-12) , pages 250-254,
- MARQUEZ-SILLERO, I. et al.: "Headspace-multicapillary column-ion mobility spectrometry for the direct analysis of 2,4,6-trichloroanisole in wine and cork samples", Journal of Chromatography A, vol. 1265, November 2012 (2012-11), pages 149-154,
- FIGUEIREDO, F.: "2,4,6-tricloroanisol em rolhas de cortiga natural : eficacia do equipamento de deteção individual na prevengao da contaminagao de vinhos", Master's thesis, February 2014 (2014-02),

## Description

La présente invention s'inscrit dans le domaine du contrôle de la qualité des bouchons en liège, destinés à fermer les bouteilles de vin. Plus particulièrement, elle concerne un procédé d'analyse d'un bouchon en liège vis-à-vis de la présence de 2,4,6-trichloroanisole selon la revendication 1 ainsi qu'un dispositif pour la mise en oeuvre d'un tel procédé. L'invention concerne également un procédé plus global de sélection, dans un lot de bouchons en liège, des bouchons sensiblement dépourvus de 2,4,6-trichloroanisole, ce procédé mettant en œuvre les étapes d'un procédé d'analyse selon l'invention.

Les bouchons en liège sont utilisés de manière extensive pour boucher des récipients contenant des produits alimentaires, plus particulièrement des bouteilles contenant des boissons, et en particulier du vin, depuis de nombreuses années. Le liège, matériau naturel qui constitue l'écorce du chêne liège Quercus suber, présente en effet des propriétés particulièrement avantageuses à cet effet, notamment des propriétés d'imperméabilité aux liquides, de légèreté et des propriétés mécaniques d'élasticité, résilience et compressibilité.

Par bouchon en liège, on entend dans la présente description aussi bien les bouchons constitués exclusivement de liège, que les bouchons en matériau à base de liège, dont le liège est un composant majoritaire.

L'utilisation de bouchons en liège pour boucher les bouteilles de liquide peut occasionnellement induire un effet négatif sur la qualité du liquide conservé, en particulier lorsque ce liquide est du vin, plus précisément des altérations de son odeur et/ou de son goût. Dans le domaine de l'oenologie, ces altérations sont communément désignées par l'expression « goût de bouchon ». Il a été démontré que le goût de bouchon est majoritairement dû à la présence dans le liège constituant le bouchon d'une molécule aromatique contaminante, le 2,4,6-trichloroanisole (TCA), qui est relarguée par le bouchon et qui interagit avec le vin pour en modifier les propriétés organoleptiques. Cette molécule provient de la méthylation de chlorophénols, pouvant être issus d'origines diverses.

Il suffit d'une dose très faible de TCA dans le vin pour que les consommateurs y détectent un goût de bouchon : le seuil de détection du TCA par le nez humain est en effet très bas, inférieur à 4 ng/l. Il est donc un enjeu important pour la filière vin de réduire au maximum la contamination par le TCA des bouchons en liège mis en oeuvre pour le bouchage des bouteilles, notamment des bouteilles de vin.

Il a été proposé par l'art antérieur diverses méthodes de traitement du liège, préalablement à sa mise en forme en bouchon, visant à le débarrasser de ses contaminants organiques aromatiques, notamment du TCA. Cependant, malgré les efforts déployés, il n'a pas été possible jusqu'à présent d'assurer une décontamination totale des bouchons en liège, en particulier vis-à-vis du TCA.

Il est préconisé aussi, avant d'utiliser les bouchons de liège pour le bouchage des bouteilles conservant un liquide alimentaire, et plus particulièrement du vin, de tester ces bouchons vis-à-vis de la présence de 2,4,6-trichloroanisole, et d'anticiper ainsi en amont le risque d'altération de la qualité du liquide lié aux bouchons. Une telle analyse est à ce jour considérée comme indispensable dans le contrôle de la qualité des bouchons en liège.

La principale technique proposée par l'art antérieur à cet effet, et objet de la norme ISO 20752 (« Bouchons en liège - Dosage du 2,4,6-trichloroanisol (TCA) relarguable »), consiste à prélever, dans un lot de bouchons, un échantillon de bouchons, typiquement composé de vingt bouchons, et à le faire macérer dans une solution simulant le vin, plus précisément une solution hydroalcoolique, de sorte à simuler les phénomènes de migration du TCA susceptibles de se produire entre les bouchons en liège et le vin. Un aliquot du macérât ainsi obtenu est prélevé par la technique de microextraction en phase solide, puis analysé par chromatographie en phase gazeuse, avec détection par spectrométrie de masse ou capture d'électrons. Une telle méthode, couramment désignée par l'abréviation SPME, est cependant longue à mettre en oeuvre. En outre, en raison de son caractère destructif des bouchons, elle ne peut être mise en oeuvre que sur un nombre réduit de bouchons d'un lot de bouchons donné. Elle ne permet donc d'obtenir qu'un résultat globalisé de contamination du lot, et nullement de garantir que chaque bouchon individuel du lot de bouchons est exempt de contamination au 2,4,6-trichloroanisole.

Il a par ailleurs été proposé dans la publication de Lorenzo et al. (2006) Journal of Chromatography, 114: 250-254, un procédé pour l'analyse d'un bouchon en liège pour la présence d'haloanisoles, notamment de 2,4,6-trichloroanisole, par des étapes successives de : chauffage du bouchon dans un flacon dans lequel est en outre disposé, au-dessus du bouchon, un barreau enrobé d'une couche de polydiméthylsiloxane (PDMS) ; adsorption sur ce barreau des vapeurs de certains composés chimiques volatiles s'échappant du bouchon lors du chauffage ; désorption thermique de ces composés chimiques ; analyse de ces composés par chromatographie en phase gazeuse couplée à la spectrométrie de masse. Un tel procédé s'avère cependant complexe, long et coûteux à mettre en oeuvre. Il ne permet en outre pas d'obtenir des résultats fiables concernant la quantité d'haloanisoles initialement contenus dans le bouchon, en raison des pertes de composés se produisant lors des étapes d'adsorption / désorption sur le barreau.

La présente invention vise à remédier aux inconvénients des procédés d'analyse des bouchons en liège vis-à-vis de la présence de 2,4,6-trichloroanisole proposés par l'art antérieur, notamment à ceux exposés ci-avant, en proposant un tel procédé qui permette une détection fiable et sensible de la présence de 2,4,6-trichloroanisole dans les bouchons en liège, et le cas échéant son dosage, tout en étant non destructif, c'est-à-dire qu'il n'altère pas les propriétés chimiques, physiques et organoleptiques du liège, de sorte à pouvoir être mis en œuvre sur chaque bouchon individuel d'un lot de bouchon, en assurant que ce bouchon reste utilisable pour le bouchage ultérieur des bouteilles de vin. L'invention vise également à ce que ce procédé puisse être mis en œuvre de manière totalement, ou au moins partiellement, automatisée, facilement, rapidement et à coût réduit.

Ainsi, selon un premier aspect, la présente invention concerne un procédé d'analyse d'un bouchon en liège destiné à boucher une bouteille de vin pour la présence de 2,4,6-trichloroanisole, qui comprend la succession des étapes suivantes :
- introduction du bouchon dans un contenant,
- fermeture hermétique du contenant,
- chauffage du contenant renfermant le bouchon dans des conditions, en particulier de température, pression et durée, permettant une vaporisation de 2,4,6-trichloroanisole éventuellement présent dans le bouchon, plus précisément d'au moins une partie du 2,4,6-trichloroanisole éventuellement présent dans le bouchon,
- prélèvement d'un échantillon gazeux de l'atmosphère environnant le bouchon dans le contenant,
- injection de l'échantillon gazeux prélevé dans le contenant (11) directement dans un module d'analyse, et
- analyse de l'échantillon gazeux ainsi prélevé pour la présence de 2,4,6-trichloroanisole.

Le procédé selon l'invention prévoit notamment avantageusement le prélèvement d'un échantillon gazeux de l'atmosphère environnant le bouchon dans le contenant, c'est-à-dire d'une fraction de l'atmosphère globale présente dans le contenant et environnant le bouchon. L'échantillon présente de ce fait une composition identique à la composition globale de l'atmosphère présente dans le contenant. L'invention diffère notamment en cela des procédés proposés par l'art antérieur qui réalisent le prélèvement sélectif, dans le contenant dans lequel est placé le bouchon, de composés particuliers présents dans l'atmosphère. Contrairement à ce que permettent de tels procédés, l'échantillon gazeux ainsi prélevé selon l'invention offre notamment l'avantage de pouvoir être utilisé directement pour l'étape ultérieure d'analyse, sans devoir subir au préalable une étape de traitement supplémentaire, si bien que le procédé selon l'invention est plus simple, plus rapide, plus fiable et moins coûteux à mettre en oeuvre.

En particulier, le procédé selon l'invention ne comporte avantageusement pas d'étape d'introduction dans le contenant d'un quelconque élément autre que le bouchon, notamment d'un barreau de PDMS.

Il ne comporte pas non plus d'étape d'introduction de composant liquide dans le contenant renfermant le bouchon, en particulier tel qu'une solution hydroalcoolique. Ainsi, le chauffage du bouchon enfermé de manière hermétique dans le contenant est réalisé à sec. Ce procédé n'est avantageusement pas destructif : sa mise en oeuvre ne provoque pas de dégradation des propriétés du liège, si bien que le bouchon ayant été soumis à ce procédé peut ensuite être utilisé de façon normale pour le bouchage des bouteilles, ceci sans traitement préalable.

Il entre dans les compétences de l'homme du métier de déterminer les combinaisons de conditions de température, pression et durée qui permettent une vaporisation de 2,4,6-trichloroanisole éventuellement présent dans le bouchon, en fonction notamment de la température de vaporisation de ce dernier, connue en elle-même, et de la rapidité souhaitée pour l'analyse du bouchon.

Selon des modes de mise en oeuvre particuliers, le procédé d'analyse selon l'invention répond en outre aux caractéristiques suivantes, mises en oeuvre séparément ou en chacune de leurs combinaisons techniquement opérantes.

Les conditions de température, pression et durée auxquelles est soumis le contenant renfermant le bouchon lors de l'étape de chauffage sont avantageusement choisies de sorte à assurer l'établissement d'un équilibre entre le 2,4,6-trichloroanisole contenu dans le bouchon et le 2,4,6-trichloroanisole sous forme gazeuse présent dans l'atmosphère environnant le bouchon dans le contenant. L'étape de prélèvement de l'échantillon gazeux dans l'atmosphère environnant le bouchon dans le contenant est alors réalisée après que cet équilibre ait été établi.

Dans des modes de mise en oeuvre particuliers de l'invention, le chauffage du contenant renfermant le bouchon est réalisé à une température comprise entre 50 et 75 °C, sous pression atmosphérique et pendant une durée comprise entre 45 et 90 minutes, de préférence d'environ 60 minutes. De telles conditions opératoires permettent avantageusement d'assurer une vaporisation de 2,4,6-trichloroanisole éventuellement contenu dans le bouchon, y compris lorsqu'il y est présent dans de faibles teneurs, tout en préservant l'intégrité et les propriétés du bouchon, et d'assurer l'établissement d'un équilibre entre le 2,4,6-trichloroanisole contenu dans le bouchon et le 2,4,6-trichloroanisole sous forme gazeuse présent dans l'atmosphère environnant le bouchon dans le contenant.

L'étape d'analyse de l'échantillon gazeux pour la présence de 2,4,6-trichloroanisole du procédé selon l'invention peut comprendre la quantification du 2,4,6-trichloroanisole présent dans cet échantillon gazeux. Cette quantification peut être absolue, par comparaison de l'échantillon avec une gamme étalon de compositions de concentrations en 2,4,6-trichloroanisole connues, ou relative.

L'étape d'analyse de l'échantillon gazeux pour la présence de 2,4,6-trichloroanisole peut être réalisée par toute technique classique en elle-même. Dans des modes de mise en oeuvre particuliers de l'invention, elle est réalisée par chromatographie en phase gazeuse couplée à une méthode de détection. L'échantillon gazeux prélevé dans le contenant est injecté directement dans le chromatographe en phase gazeuse, c'est-à-dire sans être soumis à une quelconque étape de traitement, notamment à une étape de désorption d'un quelconque élément.

La méthode de détection couplée à la chromatographie en phase gazeuse est classique en elle-même. Elle peut notamment être choisie parmi la spectrométrie de masse, la capture d'électrons, la spectrométrie de mobilité ionique et la spectrométrie de masse à mobilité ionique, de telles méthodes présentant les avantages d'une mise en oeuvre rapide, fiable, et d'une haute sensibilité de détection.

La séparation des composants contenus dans l'échantillon gazeux par chromatographie en phase gazeuse peut notamment être réalisée au moyen d'une colonne de polydiméthylsiloxane. Les conditions de chromatographie peuvent être aisément déterminées par l'homme du métier, en fonction notamment de la rapidité avec laquelle il souhaite que soit réalisée l'analyse.

De manière tout à fait avantageuse, dans le cas où l'étape d'analyse met en oeuvre un chromatographe en phase gazeuse, le prélèvement d'échantillon gazeux dans le contenant renfermant le bouchon à analyser est préférentiellement réalisé directement par la seringue du système d'injection du chromatographe, pour injection directe en tête de colonne. A cet effet, l'étape préalable de fermeture hermétique du contenant dans lequel a été placé le bouchon met en oeuvre des moyens de fermeture équipés d'une paroi apte à être transpercée par l'aiguille de la seringue, par exemple d'un septum.

Préférentiellement le procédé d'analyse selon l'invention est mis en oeuvre de manière partiellement ou totalement automatisée. En particulier, les étapes de chauffage, de prélèvement de l'échantillon gazeux, et d'analyse sont réalisées de manière automatique, et l'approvisionnement en échantillon des moyens de chauffage, des moyens de prélèvement et des moyens d'analyse est également réalisé de manière automatique.

Le procédé selon l'invention présente avantageusement une sensibilité de détection au moins aussi bonne que celle des procédés proposés par l'art antérieur, notamment du procédé SPME. Le procédé selon l'invention permet notamment d'atteindre un seuil de détection du 2,4,6-trichloroanisole équivalent à une valeur de 0,5 ng/l obtenue par le procédé SPME, et un seuil de quantification du 2,4,6-trichloroanisole équivalent à une valeur de 1,0 ng/l obtenue par le procédé SPME.

Selon un autre aspect, la présente invention concerne un procédé plus global de sélection, dans un lot de bouchons en liège, de bouchons sensiblement dépourvus de 2,4,6-trichloroanisole, qui comprend la succession des étapes suivantes :
- mise en oeuvre, pour chacun des bouchons du lot, d'un procédé d'analyse selon l'invention, répondant à l'une ou plusieurs des caractéristiques ci-avant,
- et sélection, dans le lot de bouchons, des bouchons pour lesquels la présence de 2,4,6-trichloroanisole n'est pas détectée.

Dans des modes de mise en oeuvre particuliers de l'invention, le procédé de sélection comprend en outre la détermination, pour chacun des bouchons du lot, d'une valeur représentative de la concentration de 2,4,6-trichloroanisole dans l'échantillon gazeux. Cette valeur représentative peut par exemple consister en l'aire d'un pic détecté, au temps de rétention correspondant au 2,4,6-trichloroanisole, par chromatographie en phase gazeuse avec détection par capture d'électrons. Pour les bouchons pour lesquels cette valeur représentative n'est pas nulle, le procédé de sélection peut alors comprendre :
- la comparaison de cette valeur représentative avec une valeur seuil prédéterminée,
- et la sélection des bouchons pour lesquels la valeur représentative est inférieure ou égale à la valeur seuil.

Un tel procédé de contrôle individuel de la contamination de l'ensemble des bouchons du lot de bouchons permet de sélectionner, pour un usage ultérieur, uniquement les bouchons dont il est garanti qu'ils sont sensiblement dépourvus de 2,4,6-trichloroanisole. On entend, par sensiblement dépourvus, le fait que les bouchons sont exempts de 2,4,6-trichloroanisole, ou contiennent une faible teneur en ce contaminant, inférieure à une valeur seuil prédéterminée en fonction de l'application particulière visée pour le lot de bouchons.

Le procédé selon l'invention permet ainsi d'élaborer une conclusion fiable et réelle en terme de distribution des bouchons selon la fréquence et la concentration de l'éventuelle contamination par le 2,4,6-trichloroanisole, contrairement à la méthode SPME proposée par l'art antérieur et objet de la norme ISO 20752, qui ne permet d'élaborer qu'une conclusion approchée avec un raisonnement statistique.

Le procédé de sélection peut en outre comporter une étape de tri des contenants, en fonction des résultats de l'analyse des échantillons gazeux qui en sont prélevés pour la présence de 2,4,6-trichloroanisole, cette étape étant de préférence réalisée de manière automatique. En particulier, cette étape peut consister en la répartition des contenants en plusieurs groupes, dont au moins un groupe pour lequel la présence de 2,4,6-trichloroanisole n'est pas détectée, et un groupe pour lequel la présence de 2,4,6-trichloroanisole est détectée. Le procédé peut également prévoir de diviser ce dernier groupe en plusieurs sous-groupes, correspondant d'une part aux bouchons pour lesquels la valeur représentative est inférieure ou égale à une valeur seuil prédéterminée, et d'autre part aux bouchons pour lesquels la valeur représentative est supérieure à ladite valeur seuil prédéterminée.

Le procédé de sélection peut également comporter une étape d'ouverture des contenants et de récupération des bouchons qui y sont contenus, de préférence également réalisée de manière automatique.

Selon un autre aspect, la présente invention concerne un dispositif pour la mise en œuvre d'un procédé d'analyse d'un bouchon en liège pour la présence de 2,4,6-trichloroanisole, et/ou d'un procédé de sélection, selon l'invention. Ce dispositif comporte :
- un module automatisé de chauffage d'un contenant dans lequel est placé un bouchon de liège destiné à boucher une bouteille de vin, dans des conditions permettant une vaporisation de 2,4,6-trichloroanisole éventuellement présent dans ledit bouchon,
- un module automatisé de prélèvement d'un échantillon gazeux de l'atmosphère environnant le bouchon dans le contenant,
- un module automatisé d'analyse de l'échantillon gazeux ainsi prélevé pour la présence de 2,4,6-trichloroanisole,
- le cas échéant, des moyens automatisés de convoyage du contenant renfermant le bouchon jusqu'au module de chauffage et/ou du module de chauffage au module de prélèvement,
- et le cas échéant, un module automatisé de tri des contenants en fonction des résultats de l'analyse pour la présence de 2,4,6-trichloroanisole.

Dans des modes de réalisation particuliers de l'invention, le dispositif comporte en outre un module automatisé d'introduction d'un bouchon dans un contenant et/ou un module de fermeture hermétique du contenant dans lequel le bouchon a été introduit.

L'ensemble des modules et les moyens de convoyage du dispositif selon l'invention sont classiques en eux-mêmes.

Le module d'analyse de l'échantillon gazeux prélevé dans le contenant pour la présence de 2,4,6-trichloroanisole peut notamment comporter un chromatographe en phase gazeuse couplé à des moyens de détection. Ces moyens de détection sont classiques en eux-mêmes, et peuvent notamment être choisis parmi les spectromètres de masse, les détecteurs à capture d'électrons, les spectromètres de mobilité ionique et les spectromètres de masse à mobilité ionique. Le module automatisé de prélèvement d'un échantillon gazeux dans le contenant comporte alors un système d'injection automatique du chromatographe en phase gazeuse.

Préférentiellement, le module automatisé de prélèvement de l'échantillon gazeux dans le contenant est le système d'injection automatique du chromatographe en phase gazeuse. Ce système d'injection réalise alors avantageusement le prélèvement de l'échantillon gazeux de l'atmosphère présente dans le contenant autour du bouchon à analyser, et l'injection de cet échantillon directement dans le chromatographe.

Dans des modes de réalisation préférés de l'invention, le dispositif comporte au moins deux modules automatisés d'analyse d'échantillons gazeux prélevés des contenants, qui sont montés en parallèle, et mis en oeuvre en alternance, de sorte à pouvoir analyser un nombre au moins deux fois plus important d'échantillons gazeux dans un même intervalle de temps.

Le dispositif peut également comporter un module d'ouverture des contenants, et d'extraction des bouchons qui y sont contenus.

Dans des modes de réalisation de l'invention particulièrement avantageux pour la mise en oeuvre industrielle du procédé selon l'invention, le dispositif comporte en outre un module de commande automatique des modules automatisés entrant dans la constitution du dispositif selon l'invention, et le cas échéant des moyens de convoyage.

En particulier, ce module de commande automatique comporte des moyens d'asservissement du module de tri des contenants aux résultats d'analyse des échantillons gazeux pour la présence de 2,4,6-trichloroanisole.

Les différentes étapes de mémorisation des informations, de comparaison des valeurs mesurées avec les valeurs seuil prédéterminées, et de commande des différents modules sont de préférence effectuées par module du type ordinateur programmé, comportant au moins un microprocesseur, et des moyens de mémorisation (disque dur magnétique, mémoire flash, disque optique, etc.) dans lesquels est mémorisé un produit programme d'ordinateur, sous la forme d'un ensemble d'instructions de code de programme à exécuter pour mettre en oeuvre les différentes étapes de calcul et de commande du procédé selon l'invention.

Un dispositif répondant à de telles caractéristiques permet avantageusement de mettre en oeuvre le procédé d'analyse selon l'invention en continu et en série sur un grand nombre de bouchons à analyser individuellement.

Les caractéristiques et avantages de l'invention apparaîtront plus clairement à la lumière de l'exemple de mise en oeuvre ci-après, fourni à simple titre illustratif et nullement limitatif de l'invention, avec l'appui des figures 1 à 3, dans lesquelles :
- la figure 1 représente de manière schématique un dispositif automatisé pour la mise en oeuvre des étapes finales d'un procédé selon l'invention d'analyse d'un bouchon en liège pour la présence de 2,4,6-trichloroanisole ;
- la figure 2a montre un exemple de chromatogramme obtenu après mise en oeuvre d'un procédé selon l'invention, d'analyse d'un bouchon en liège pour la présence de 2,4,6-trichloroanisole ;
- la figure 2b montre un chromatogramme obtenu pour une solution de 2,4,6-trichloroanisole à 2 ng/l dans l'éthanol, analysée dans les mêmes conditions que pour l'obtention du chromatogramme de la figure 2a ;
- et la figure 3 montre un graphe représentant, pour 10 bouchons analysés individuellement, successivement par un procédé conforme à l'invention et par la méthode SPME de l'art antérieur, l'aire sous le pic mesurée au temps de rétention du 2,4,6-trichloroanisole par mise en oeuvre du procédé selon l'invention, en fonction de la concentration en 2,4,6-trichloroanisole mesurée par la méthode SPME.

Un procédé selon l'invention d'analyse d'un bouchon en liège pour la présence de 2,4,6-trichloroanisole comprend les étapes suivantes.

En première étape, un bouchon en liège est introduit dans un contenant 11, par exemple d'une contenance de 100 ml.

Ce contenant 11 est fermé de manière hermétique, notamment au moyen d'un obturateur approprié au système d'injection d'un chromatographe en phase gazeuse.

Ces premières étapes peuvent être réalisées de manière manuelle ou automatisée, par des modules automatiques adéquats classiques en eux-mêmes pour l'introduction en série d'articles solides dans des contenants individuels et la fermeture de ces contenants.

Le contenant 11 renfermant un bouchon est ensuite soumis à une étape de chauffage, à une température comprise entre 50 et 75 °C, plus particulièrement à 70 °C, sous pression atmosphérique, pendant 1 h. Ces conditions de chauffage permettent de provoquer le relargage d'au moins une partie du 2,4,6-trichloroanisole susceptible d'être contenu dans le liège, sous forme gazeuse, dans l'atmosphère interne du contenant 11, et la stabilisation dans un état d'équilibre.

Un échantillon, par exemple de 0,5 ml, de l'atmosphère présente dans le contenant 11 contenant le bouchon, est ensuite prélevé du contenant 11 et analysé pour la présence de 2,4,6-trichloroanisole.

Cette analyse est par exemple effectuée par chromatographie en phase gazeuse et détection par capture d'électrons.

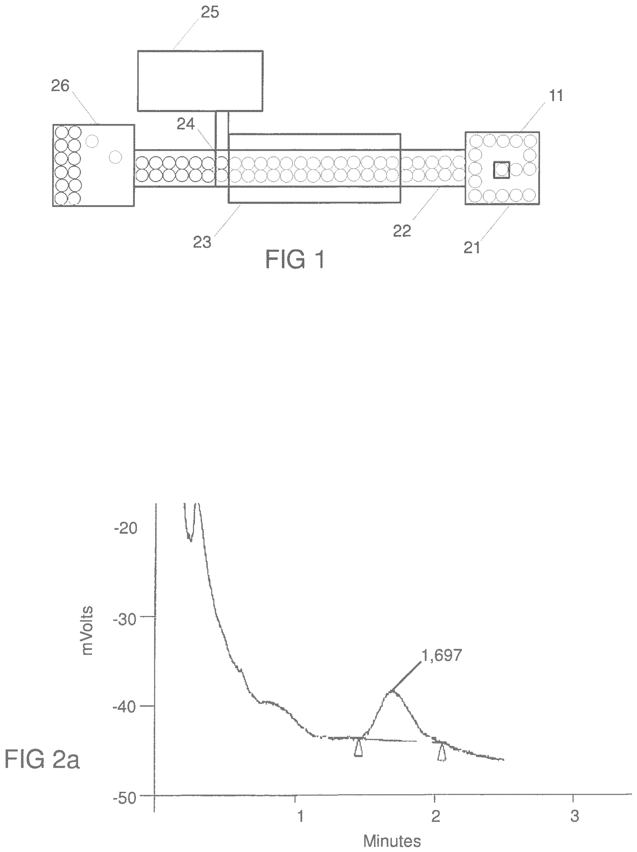

Un exemple de dispositif pour la mise en oeuvre en série et de manière automatisée de ces étapes du procédé selon l'invention est représenté de façon schématique sur la figure 1.

Ce dispositif comporte un module 21 de réception des contenants 11 renfermant chacun un bouchon en liège.

Depuis ce module de réception 21, les contenants 11 sont transportés, par un convoyeur 22, classique en lui-même, à travers un tunnel de chauffage 23. Ce tunnel de chauffage peut être de tout type classique en lui-même.

La puissance de chauffage du tunnel de chauffage 23 et la vitesse du convoyeur 22 sont réglées de sorte à ce que chaque contenant 11 soit soumis, dans le tunnel de chauffage 23, à une température comprise entre 50 et 75 °C pendant 1 h.

En sortie du tunnel de chauffage 23, le contenant 11 est conduit, toujours par le convoyeur 22, jusqu'à un module 24 de prélèvement d'un échantillon gazeux de l'atmosphère interne du contenant 11 environnant le bouchon. Ce prélèvement est préférentiellement réalisé par le système d'injection automatique d'un chromatographe en phase gazeuse. Sur la figure 1, la référence 25 désigne l'ensemble de ce chromatographe et d'un détecteur associé.

L'échantillon gazeux ainsi prélevé est injecté dans le chromatographe, et analysé pour la présence de 2,4,6-trichloroanisole.

Au sortir du module de prélèvement 24, le contenant 11, renfermant toujours le bouchon, est transporté par le convoyeur 22 jusqu'à un module 26 de collecte des contenants, en vue de leur tri en fonction des résultats de l'analyse par chromatographie en phase gazeuse.

Le dispositif peut également comporter un module de tri automatisé des contenants, en fonction des résultats de l'analyse pour la présence de 2,4,6-trichloroanisole réalisée par le chromatographe en phase gazeuse et le détecteur associé. Ce module de tri, qui n'est pas représenté sur les figures, est avantageusement commandé par un module de commande que comporte le dispositif. Le module de commande est notamment apte à acquérir les données relevées par le détecteur pour chaque contenant 11, à les traiter, et à émettre, pour chaque contenant 11, un signal de commande en direction module de tri, établi en fonction du résultat de l'analyse pour la présence de 2,4,6-trichloroanisole, de sorte à assurer une séparation des contenants 11 en fonction de la présence de 2,4,6-trichloroanisole dans les bouchons qu'ils contiennent, et/ou de la concentration en TCA mesurée.

Le chromatographe en phase gazeuse 25 est classique en lui-même.

Il est par exemple équipé de deux voies analytiques, chacune étant composée d'un injecteur, d'une vanne d'injection, d'une colonne capillaire courte et d'un détecteur, par exemple à capture d'électrons. Ceci permet avantageusement de réaliser deux analyses simultanément. Préférentiellement, les deux voies analytiques sont gérées par le même module de commande.

A titre d'exemple, un bouchon de liège quelconque a été soumis aux étapes ci-avant. L'échantillon gazeux prélevé du contenant 11 le renfermant a été analysé par chromatographie en phase gazeuse, sur une colonne de polydiméthylsiloxane d'épaisseur de phase 5 µm, couplée à un détecteur par capture d'électrons. Une solution étalon à 2 ng/L de 2,4,6-trichloroanisole dans l'éthanol a également été réalisée et injectée dans le chromatographe, dans les mêmes conditions opératoires.

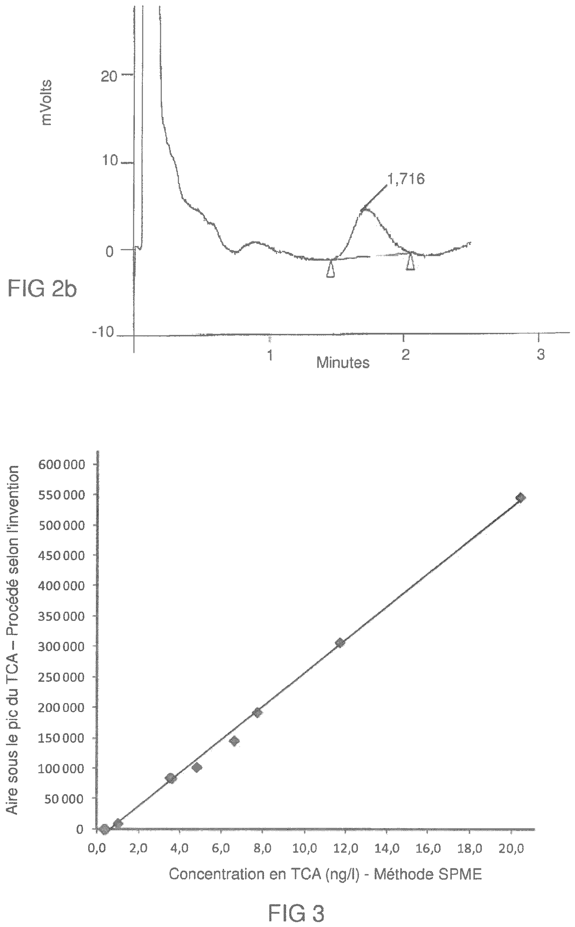

Les chromatogrammes obtenus sont montrés sur la figure 2a pour l'échantillon traité conformément à l'invention, et sur la figure 2b pour la solution étalon de 2,4,6-trichloroanisole.

On observe sur ces figures que le chromatogramme obtenu selon l'invention présente un pic à 1,697 minutes, qui peut bien être attribué au 2,4,6-trichloroanisole, dont le chromatogramme témoin montre un temps de rétention de 1,716 minutes.

Dans un procédé plus global de sélection, dans un lot de bouchons en liège, des bouchons sensiblement dépourvus de 2,4,6-trichloroanisole, ce bouchon est écarté car contaminé par le 2,4,6-trichloroanisole et donc susceptible de conférer au vin un goût de bouchon.

La quantification de la teneur en 2,4,6-trichloroanisole du bouchon peut par exemple être réalisée par mesure de l'aire sous le pic obtenu au temps de rétention correspondant au 2,4,6-trichloroanisole, et comparaison avec une gamme étalon de compositions de concentrations connues en 2,4,6-trichloroanisole.

Une analyse comparative par chromatographie en phase gazeuse couplée à la spectrométrie de masse est également réalisée sur un échantillon gazeux prélevé dans le contenant conformément à la présente invention, et sur un témoin de 2,4,6-trichloroanisole pur. Le spectromètre de masse fonctionne en mode MS/MS, avec une température d'enceinte de 45 °C et une température de l'analyseur trappe d'ion de 150 °C. On obtient des spectres de masse identiques pour l'échantillon obtenu conformément à l'invention et pour le témoin 2,4,6-trichloroanisole, ce qui confirme que le procédé selon l'invention permet de détecter, et de quantifier, le 2,4,6-trichloroanisole présent dans le bouchon de liège. Plus précisément, on retrouve dans les deux spectres un pic à 197 m/z, correspondant à la fragmentation de l'ion père à 212 m/z du 2,4,6-trichloroanisole.

La quantification de la teneur en 2,4,6-trichloroanisole du bouchon peut par exemple être réalisée par mesure de l'intensité de ce pic à 197 m/z, et comparaison avec une gamme étalon de compositions de concentrations connues en 2,4,6-trichloroanisole.

Une analyse comparative a également été réalisée avec la méthode SPME proposée par l'art antérieur. A cet effet, dix bouchons en liège ont été soumis au procédé d'analyse selon l'invention, tel que décrit ci-avant. Pour chacun, l'échantillon gazeux a été analysé par chromatographie en phase gazeuse couplée à une détection par capture d'électrons. L'aire du pic correspondant au 2,4,6-trichloroanisole a été mesurée.

Les bouchons ont ensuite été récupérés, et ont été soumis chacun au procédé SPME, en suivant le protocole classique décrit dans la norme ISO 20752. La concentration en 2,4,6-trichloroanisole, exprimée en ng/l, a été mesurée.

Les résultats obtenus, pour chacun des bouchons, par chacune des techniques sont indiqués dans le tableau 1 ci-après.

**Tableau 1 - Résultat de l'analyse de la teneur de bouchons en liège en 2,4,6-trichloroanisole, par la méthode SPME de l'art antérieur et par le procédé selon l'invention**

| Bouchon | Procédé SPME (ng/l) | Procédé selon l'invention (aire du pic) |
|---|---|---|
| 1 | 0,3 | 0 |
| 2 | 0,4 | 0 |
| 3 | 1,0 | 9 425 |
| 4 | 3,5 | 84 429 |
| 5 | 3,6 | 83 334 |
| 6 | 4,8 | 101 827 |
| 7 | 6,6 | 145 207 |
| 8 | 7,7 | 191 720 |
| 9 | 11,7 | 306 605 |
| 10 | 20,4 | 545 914 |

A partir des valeurs ainsi obtenues, il a été tracé une courbe représentant, pour chaque bouchon, l'aire sous le pic

Les valeurs obtenues pour chaque bouchon ont été reportées sur un graphe, la concentration obtenue par la méthode SPME figurant sur l'axe des abscisses et l'aire sous le pic obtenue conformément à l'invention étant représentée en ordonnée. Le graphe obtenu est montré sur la figure 3. On y observe clairement une parfaite concordance entre les valeurs obtenues par le procédé selon l'invention et celles obtenues par la méthode SPME de l'art antérieur.

Le procédé d'analyse selon l'invention permet ainsi de quantifier le 2,4,6-trichloroanisole présent dans un bouchon en liège de manière aussi fiable que la méthode SPME.

Il présente en outre de nombreux avantages par rapport aux procédés de l'art antérieur, et notamment à la méthode SPME. Il permet notamment d'analyser jusqu'à 1440 bouchons en 24 heures pour la présence de 2,4,6-trichloroanisole. Il comporte peu d'étapes, toutes réalisables facilement, rapidement et qui plus est de manière automatisée. Il n'utilise ni consommables, ni réactifs, tels qu'une solution de macération ou des fibres de microextraction en phase solide.

Enfin et surtout, il permet d'analyser chaque bouchon d'un lot individuellement, sans altérer les propriétés de ce bouchon. Il permet ainsi de garantir que chaque bouchon qu'il permet de sélectionner est exempt, ou sensiblement exempt de 2,4,6-trichloroanisole, et sera sans incidence sur le goût et/ou l'odeur du vin conservé dans la bouteille que ce bouchon servira à boucher.

## Revendications

1. Procédé d'analyse non-destructif d'un bouchon en liège destiné à boucher une bouteille de vin pour la présence de 2,4,6-trichloroanisole, comprenant la succession des étapes suivantes :
- introduction dudit bouchon dans un contenant (11),
- fermeture hermétique dudit contenant (11),
- chauffage dudit contenant (11) renfermant ledit bouchon dans des conditions permettant une vaporisation de 2,4,6-trichloroanisole éventuellement présent dans ledit bouchon,
- prélèvement d'un échantillon gazeux de l'atmosphère environnant ledit bouchon dans ledit contenant (11),
- et analyse dudit échantillon gazeux pour la présence de 2,4,6-trichloroanisole,
**caractérisé en ce que** l'échantillon gazeux prélevé dans le contenant (11) est injecté directement dans le module d'analyse.

2. Procédé d'analyse selon la revendication 1, selon lequel le chauffage du contenant (11) renfermant le bouchon est réalisé à une température comprise entre 50 et 75 °C, sous pression atmosphérique et pendant une durée comprise entre 45 et 90 minutes.

3. Procédé d'analyse selon la revendication 2, selon lequel le chauffage du contenant (11) renfermant le bouchon est réalisé à une température comprise entre 50 et 75 °C, sous pression atmosphérique et pendant une durée d'environ 60 minutes.

4. Procédé d'analyse selon l'une quelconque des revendications 1 à 3, selon lequel l'analyse de l'échantillon gazeux pour la présence de 2,4,6-trichloroanisole comprend la quantification du 2,4,6-trichloroanisole présent dans ledit échantillon gazeux.

5. Procédé d'analyse selon l'une quelconque des revendications 1 à 4, selon lequel l'analyse de l'échantillon gazeux pour la présence de 2,4,6-trichloroanisole est réalisée par chromatographie en phase gazeuse couplée à une méthode de détection, l'échantillon gazeux prélevé dans le contenant (11) étant injecté directement dans le chromatographe en phase gazeuse.

6. Procédé d'analyse selon la revendication 5, selon lequel la méthode de détection est choisie parmi la spectrométrie de masse, la capture d'électrons, la spectrométrie de mobilité ionique et la spectrométrie de masse à mobilité ionique.

7. Procédé d'analyse selon l'une quelconque des revendications 1 à 6, mis en œuvre de manière automatisée.

8. Procédé de sélection, dans un lot de bouchons en liège, de bouchons sensiblement dépourvus de 2,4,6-trichloroanisole, **caractérisé en ce qu'**il comprend la succession des étapes suivantes :
- mise en œuvre, pour chacun des bouchons dudit lot, d'un procédé d'analyse selon l'une quelconque des revendications 1 à 7,
- et sélection des bouchons pour lesquels la présence de 2,4,6-trichloroanisole n'est pas détectée.

9. Procédé de sélection selon la revendication 8, comprenant la détermination, pour chacun des bouchons du lot, d'une valeur représentative de la concentration de 2,4,6-trichloroanisole dans l'échantillon gazeux, et, pour les bouchons pour lesquels ladite valeur représentative n'est pas nulle :
- la comparaison de ladite valeur représentative avec une valeur seuil prédéterminée,
- et la sélection des bouchons pour lesquels ladite valeur représentative est inférieure ou égale à ladite valeur seuil.

10. Dispositif pour la mise en œuvre d'un procédé d'analyse selon l'une quelconque des revendications 1 à 7 et/ou d'un procédé de sélection selon l'une quelconque des revendications 8 à 9, comportant :
- un module automatisé (23) de chauffage d'un contenant (11) dans lequel est placé un bouchon de liège destiné à boucher une bouteille de vin dans des conditions permettant une vaporisation de 2,4,6-trichloroanisole éventuellement présent dans ledit bouchon,
- un module automatisé (24) de prélèvement d'un échantillon gazeux de l'atmosphère environnant ledit bouchon dans ledit contenant (11),
- un module automatisé (25) d'analyse dudit échantillon gazeux pour la présence de 2,4,6-trichloroanisole,
**caractérisé en ce que** le module automatisé (24) de prélèvement de l'échantillon gazeux comporte un système d'injection automatique pour l'injection de l'échantillon directement dans le module d'analyse (25).

11. Dispositif selon la revendication 10, comportant des moyens automatisés (22) de convoyage dudit contenant (11) audit module de chauffage (23).

12. Dispositif selon l'une quelconque des revendications 10 à 11, comportant des moyens automatisés (22) de convoyage dudit contenant (11) dudit module de chauffage (23) audit module de prélèvement (24).

13. Dispositif selon l'une quelconque des revendications 10 à 12, comportant un module automatisé de tri des contenants (11) en fonction des résultats de l'analyse pour la présence de TCA.

14. Dispositif selon l'une quelconque des revendications 10 à 13, dans lequel le module (25) d'analyse dudit échantillon gazeux pour la présence de 2,4,6-trichloroanisole comporte un chromatographe en phase gazeuse couplé à des moyens de détection, et le module automatisé (24) de prélèvement d'un échantillon gazeux dans ledit contenant (11) comporte un système d'injection automatique dudit chromatographe en phase gazeuse.

15. Dispositif selon l'une quelconque des revendications 10 à 14, comportant un module de commande automatique desdits modules automatisés (23, 24, 25) et le cas échéant desdits moyens de convoyage (22).

## Patentansprüche

1. Verfahren zur nicht-destruktiven Analyse eines Stopfens aus Kork zum Verschliessen eine Weinflasche, auf das Vorhandensein von 2,4,6-Trichloranisol, das die folgenden aufeinanderfolgenden Schritte umfasst:
- Einbringen des Stopfens in einen Behälter (11),
- hermetisches Verschließen des Behälters (11),
- Erhitzen des Behälters (11), der den Stopfen enthält, unter Bedingungen, die eine Verdampfung von gegebenenfalls in dem Stopfen vorhandenem 2,4,6-Trichloranisol gestatten,
- Entnehmen einer gasförmigen Probe aus der Atmosphäre, die den Stopfen in dem Behälter (11) umgibt,
- und Analysieren der gasförmigen Probe auf das Vorhandensein von 2,4,6-Trichloranisol,
**dadurch gekennzeichnet, dass** die im Behälter (11) genommene gasförmige Probe direkt in das Analysemodul injiziert wird.

2. Analyseverfahren nach Anspruch 1, wobei das Erhitzen des den Stopfen enthaltenden Behälters (11) bei einer Temperatur zwischen 50 und 75°C unter Atmosphärendruck und für eine Dauer zwischen 45 und 90 Minuten durchgeführt wird.

3. Analyseverfahren nach Anspruch 2, wobei das Erhitzen des den Stopfen enthaltenden Behälters (11) bei einer Temperatur zwischen 50 und 75°C unter Atmosphärendruck und für eine Dauer von etwa 60 Minuten durchgeführt wird.

4. Analyseverfahren nach einem der Ansprüche 1 bis 3, wobei die Analyse der gasförmigen Probe auf das Vorhandensein von 2,4,6-Trichloranisol die Quantifizierung von in der gasförmigen Probe vorhandenem 2,4,6-Trichloranisol umfasst.

5. Analyseverfahren nach einem der Ansprüche 1 bis 4, wobei die Analyse der gasförmigen Probe auf das Vorhandensein von 2,4,6-Trichloranisol mittels Gaschromatographie, gekoppelt mit einem Nachweisverfahren, erfolgt, wobei die im Behälter (11) genommene gasförmige Probe direkt in den Gaschromatographen injiziert wird.

6. Analyseverfahren nach Anspruch 5, wobei das Nachweisverfahren aus Massenspektrometrie, Einfangen von Elektronen, Ionenmobilitätsspektrometrie und Massenspektrometrie-Ionenmobilitätsspektrometrie ausgewählt ist.

7. Analyseverfahren nach einem der Ansprüche 1 bis 6, das automatisiert durchgeführt wird.

8. Verfahren zum Auswählen, aus einer Charge von Korkstopfen, der Stopfen, die im Wesentlichen frei von 2,4,6-Trichloranisol sind, **dadurch gekennzeichnet, dass** es die folgenden aufeinanderfolgenden Schritte umfasst:
- Durchführen eines Analyseverfahrens nach einem der Ansprüche 1 bis 7 für jeden Stopfen der Charge
- und Auswählen der Stopfen, bei denen das Vorhandensein von 2,4,6-Trichloranisol nicht nachgewiesen wird.

9. Auswahlverfahren nach Anspruch 8, umfassend das Bestimmen, für jeden der Stopfen der Charge, eines Werts, der für die Konzentration von 2,4,6-Trichloranisol in der gasförmigen Probe repräsentativ ist, und für die Stopfen, bei denen der repräsentative Wert nicht null ist:
- Vergleichen des repräsentativen Werts mit einem zuvor festgelegten Schwellenwert
- und Auswählen der Stopfen, bei denen der repräsentative Wert kleiner als der oder gleich dem Schwellenwert ist.

10. Vorrichtung zur Durchführung eines Analyseverfahrens nach einem der Ansprüche 1 bis 7 und/oder eines Auswahlverfahrens nach einem der Ansprüche 8 bis 9, die Folgendes enthält:
- ein automatisiertes Modul (23) zum Erhitzen eines Behälters (11), in dem ein Stopfen aus Kork zum Verschliessen eine Weinflasche platziert ist, unter Bedingungen, die eine Verdampfung von gegebenenfalls in dem Stopfen vorhandenem 2,4,6-Trichloranisol gestatten,
- ein automatisiertes Modul (24) zur Entnahme einer gasförmigen Probe aus der Atmosphäre, die den Stopfen in dem Behälter (11) umgibt,
- ein automatisiertes Modul (25) zum Analysieren der gasförmigen Probe auf das Vorhandensein von 2,4,6-Trichloranisol, **dadurch gekennzeichnet, dass** das automatisierte Modul (24) zur Entnahme der gasförmigen Probe ein automatisches Injektionssystem für die Injektion der Probe direkt in das Analysemodul (25) enthält.

11. Vorrichtung nach Anspruch 10, die automatisierte Mittel (22) zum Transportieren des Behälters (11) zum Erhitzungsmodul (23) enthält.

12. Vorrichtung nach einem der Ansprüche 10 bis 11, die automatisierte Mittel (22) zum Transportieren des Behälters (11) vom Erhitzungsmodul (23) zum Probenentnahmemodul (24) enthält.

13. Vorrichtung nach einem der Ansprüche 10 bis 12, die ein automatisiertes Modul zum Aussortieren der Behälter (11) in Abhängigkeit von den Ergebnissen der Analyse auf das Vorhandensein von TCA enthält.

14. Vorrichtung nach einem der Ansprüche 10 bis 13, wobei das Modul (25) zum Analysieren der gasförmigen Probe auf das Vorhandensein von 2,4,6-Trichloranisol einen Gaschromatographen enthält, der mit Nachweismitteln gekoppelt ist, und das automatisierte Modul (24) zur Entnahme einer gasförmigen Probe in dem Behälter (11) ein System für die automatische Injektion in den Gaschromatographen enthält.

15. Vorrichtung nach einem der Ansprüche 10 bis 14, die ein automatisches Steuermodul für die automatisierten Module (23, 24, 25) und gegebenenfalls Transportmittel (22) enthält.

## Claims

1. An analysis process for non-destructively analyzing a cork stopper intended for stoppering a wine bottle for the presence of 2,4,6-trichloroanisole, comprising the succession of the following steps:
- introduction of said stopper into a container (11),
- hermetic closure of said container (11),
- heating of said container (11) containing said stopper under conditions allowing vaporization of 2,4,6-trichloroanisole that might be present in said stopper,
- collection of a gaseous sample of the atmosphere surrounding said stopper in said container (11), and
- and analysis of said gaseous sample for the presence of 2,4,6-trichloroanisole,
**characterized in that** the gaseous sample collected in the container (11) is injected directly into the analysis module.

2. The analysis process as claimed in claim 1, in which the heating of the container (11) containing the stopper is performed at a temperature of between 50 and 75°C, at atmospheric pressure and for a time of between 45 and 90 minutes.

3. The analysis process as claimed in claim 2, in which the heating of the container (11) containing the stopper is performed at a temperature of between 50 and 75°C, at atmospheric pressure and for a time of about 60 minutes.

4. The analysis process as claimed in any one of claims 1 to 3, in which the analysis of the gaseous sample for the presence of 2,4,6-trichloroanisole comprises the quantification of the 2,4,6-trichloroanisole present in said gaseous sample.

5. The analysis process as claimed in any one of claims 1 to 4, in which the analysis of the gaseous sample for the presence of 2,4,6-trichloroanisole is performed by gas chromatography coupled to a detection method, the gaseous sample collected in the container (11) being injected directly into the gas chromatograph.

6. The analysis process as claimed in claim 5, in which the detection method is chosen from mass spectrometry, electron uptake, ion mobility spectrometry and ion mobility mass spectrometry.

7. The analysis process as claimed in any one of claims 1 to 6, performed in an automated manner.

8. A selection process for selecting, in a batch of cork stoppers, stoppers that are substantially free of 2,4,6-trichloroanisole, **characterized in that** it comprises the succession of the following steps:
- implementation, for each of the stoppers of said batch, of an analysis process as claimed in any one of claims 1 to 7, and
- selection of the stoppers for which the presence of 2,4,6-trichloroanisole is not detected.

9. The selection process as claimed in claim 8, comprising the determination, for each of the stoppers of the batch, of a value representative of the concentration of 2,4,6-trichloroanisole in the gaseous sample, and, for the stoppers for which said representative value is not zero:
- comparison of said representative value with a predetermined threshold value, and
- selection of the stoppers for which said representative value is less than or equal to said threshold value.

10. A device for performing an analysis process as claimed in any one of claims 1 to 7 and/or a selection process as claimed in either of claims 8 and 9, comprising:
- an automated heating module (23) for heating a container (11) in which is placed a cork stopper intended for stoppering a wine bottle under conditions allowing vaporization of any 2,4,6-trichloroanisole present in said stopper,
- an automated sample collection module (24) for collecting a gaseous sample of the atmosphere surrounding said stopper in said container (11),
- an automated analysis module (25) for analyzing said gaseous sample for the presence of 2,4,6-trichloroanisole, **characterized in that** the automated sample collection module (24) for collecting the gaseous sample comprises an automatic injection system for injecting the sample directly in the analysis module (25).

11. The device as claimed in claim 10, comprising automated means (22) for conveying said container (11) to said heating module (23).

12. The device as claimed in either one of claims 10 and 11, comprising automated means (22) for conveying said container (11) from said heating module (23) to said sample collection module (24).

13. The device as claimed in any one of claims 10 to 12, comprising an automated module for sorting the containers (11) according to the results of the analysis for the presence of TCA.

14. The device as claimed in any one of claims 10 to 13, in which the analysis module (25) for analyzing said gaseous sample for the presence of 2,4,6-trichloroanisole comprises a gas chromatograph coupled to detection means, and the automated sample collection module (24) for collecting a gaseous sample in said container (11) comprises an automatic injection system of said gas chromatograph.

15. The device as claimed in any one of claims 10 to 14, comprising a module for the automatic control of said automated modules (23, 24, 25) and, where appropriate, of said conveying means (22).
